# EUROPÄISCHE PATENTSCHRIFT

(11) **EP 1 570 810 B1**
(45) Veröffentlichungstag und Bekanntmachung des Hinweises auf die Patenterteilung: **03.01.2007**
(21) Anmeldenummer: 04004256.6
(22) Anmeldetag: 25.02.2004
(51) Int. Cl.: A61F 2/34, A61F 2/30

(54) **Beckenteilendoprothese**
Partial pelvic endoprothesis
Endoprothèse partielle du bassin

(43) Veröffentlichungstag der Anmeldung: 07.09.2005
(73) Patentinhaber: Zimmer GmbH, 8404 Winterthur (CH)
(72) Erfinder: Karrer, David, 8610 Uster (CH); Gross, Walter, 8475 Ossingen (CH)
(74) Vertreter: Manitz, Finsterwald & Partner GbR

(56) Entgegenhaltungen:
- EP-A- 0 628 294
- DE-C- 4 133 433
- US-A- 5 507 825
- US-A- 5 916 268

## Beschreibung

Die Erfindung betrifft eine Beckenteilendoprothese mit wenigstens einem an einem reserzierten Darmbeinknochen befestigbaren Basiselement und zumindest einer in einem Abstand dazu anbringbaren Hüftschale, wobei das Basiselement einen ersten Ansatz und die Hüftschale einen zweiten Ansatz aufweist.

Es gibt Tumorpatienten, bei denen das Darmbein, d.h. der halbe Beckenknochen, um ein Drittel bis zwei Drittel seiner Höhe von unten reserziert werden muss. Ohne weitere Hilfsmaßnahmen war es früher üblich, das zugehörige Bein zu amputieren, um Komplikationen wegen fehlender Befestigung am Rumpf zu vermeiden. Um derartige Amputationen zu vermeiden, sind Beckenteilendoprothesen entwickelt worden, die nicht übermäßig belastet werden dürfen, aber zumindest eine passive Platzhalterfunktion für das daran befestigte Bein ausüben.

Eine derartige Endoprothese ist in der Patentanmeldung WO 88/01491 gezeigt, in welcher an einer Hüftschale zwei Ansätze angeformt sind, welche jeweils eine Aufnahmebohrung für einen Zapfen mit selbsthemmendem Festsitz aufweisen. Die Zapfen sind an ihrem anderen Ende jeweils am reserzierten Darmbein oder Schambein verankert. Die Verankerung am Darmbein erfolgt dabei über ein plattenförmiges Basiselement.

Eine weitere Endoprothese dieser Art ist aus der Patentanmeldung EP 0 628 294 A 1 bekannt. An einem Ansatz einer Hüftschale sind Aufnahmebohrungen für Zapfen mit einem selbsthemmenden Sitz vorgesehen. Die Befestigung eines den Zapfen fortsetzenden Stielteils erfolgt durch intramedulläres Eingreifen in eine in den Darmbeinstumpf gefräste Nut. Zusätzlich ist eine mechanische Verstelleinheit vorgesehen, mit Hilfe derer das Stielteil in einem Langloch eines am Darmbein befestigten Basiselements verstellt werden kann.

Nachteilig an den vorstehend genannten Beckenteilendoprothesen ist jedoch, dass die Länge des Zapfens und die Winkellage des Zapfens relativ zum Basiselement und somit auch die Position und Ausrichtung der Hüftschale fest vorgegeben sind. Die Solllage der Hüftschale muss daher bereits bei der Resektion des Darmbeinknochens hinsichtlich Höhe und Richtung des Resektionsschnitts berücksichtigt werden.

EP 0 628 294 A1 betrifft eine Beckenteilendoprothese mit einer Hüftschale, die einen Ansatz aufweist, in dem zwei Aufnahmebohrungen ausgebildet sind, und einem Basiselement, das an dem Darmbeinbereich des Restbeckens befestigbar ist. US 5,916,268 betrifft eine Beckenteilendoprothese mit einer Hüftschale und Abstützvorrichtungen zur Abstützung der Hüftschale am Beckenknochen. Die Abstützvorrichtungen können Abstandshalter oder Zwischenstücke verschiedener Dicke oder Länge umfassen.

Der Erfindung liegt die Aufgabe zugrunde, eine Beckenteilendoprothese der eingangs genannten Art zu schaffen, die sich durch einen möglichst einfachen Aufbau bei gleichzeitig erhöhter Flexibilität hinsichtlich der Positionierung und/oder Ausrichtung der Hüftschale auszeichnet.

Die Lösung dieser Aufgabe erfolgt durch die Merkmale des Anspruchs 1

Bei der erfindungsgemäßen Beckenteilendoprothese ist vorgesehen, dass zwischen dem Ansatz des Basiselements und dem Ansatz der Hüftschale ein weiteres Bauteil einbaubar ist. Durch die Möglichkeit, ein separates Zwischenelement einzubauen, ist eine erhöhte Flexibilität der Beckenteilendoprothese gewährleistet. Diese Flexibilität ist insbesondere dann von Vorteil, wenn der Operateur während des operativen Eingriffs Verhältnisse vorfindet, die einen von der ursprünglichen Operationsplanung abweichenden Resektionsschnitt erfordern.

Vorteilhafte Ausführungsformen der Erfindung sind in den Unteransprüchen, der Beschreibung sowie der Zeichnung angegeben.

Vorzugsweise sind mittels des Zwischenelements unterschiedliche Relativstellungen zwischen dem Basiselement und der Hüftschale realisierbar. Dies ermöglicht insbesondere, dass auch noch nach der Verankerung des Basisteils am Darmbeinknochen unterschiedliche Stellungen der Hüftschale realisierbar sind.

Besonders vorteilhaft ist es, wenn wenigstens zwei voneinander verschiedene Zwischenelemente vorgesehen sind, die jeweils zwischen dem ersten Ansatz und dem zweiten Ansatz einbaubar sind. Die erfindungsgemäße Beckenteilendoprothese stellt hierdurch einen Baukasten unterschiedlicher Zwischenelemente bereit, die alternativ zueinander zwischen dem ersten Ansatz des Basiselements und dem zweiten Ansatz der Hüftschale einbaubar sind. Der die Beckenteilendoprothese einsetzende Operateur kann aus einem Satz voneinander verschiedener Zwischenelemente dasjenige Zwischenelement auswählen, das die für den jeweiligen Patienten günstigste Sollstellung der Hüftschale ermöglicht.

Bevorzugt sind Zwischenelemente unterschiedlicher Länge vorgesehen, so dass der Abstand zwischen dem Basiselement und der Hüftschale und somit der Abstand zwischen dem reserzierten Darmbeinstumpf und der Gelenkkugel des Oberschenkelknochens variiert werden kann.

Zusätzlich oder alternativ kann wenigstens ein Zwischenelement gekröpft sein, so dass die Mittenachsen des ersten Ansatzes und des zweiten Ansatzes gegeneinander verkippt oder parallel versetzt verlaufen können. Hierdurch werden weitere Relativstellungen zwischen dem Basiselement und der Hüftschale ermöglicht, die durch bloße Verlängerung mittels eines geraden Zwischenelements nicht realisierbar wären.

Ferner kann erfindungsgemäß vorgesehen sein, dass das Zwischenelement und der erste und zweite Ansatz in Kontaktflächen jeweils mit Vor- und Rücksprüngen versehen sind, die in verschiedenen Winkelstellungen einrastbar sind. Insbesondere können die Vor- und Rücksprünge in den Kontaktflächen einer Hirth-Verzahnung entsprechen. Hierdurch wird erreicht, dass insbesondere bei Verwendung eines gekröpften Zwischenelements und/oder einer bezüglich ihrer Polachse nicht rotationssymmetrisch ausgebildeten Hüftschale diese, insbesondere deren Polachse, in verschiedene Raumrichtungen orientiert werden kann, so dass wiederum weitere Relativstellungen zwischen dem Basiselement und der Hüftschale realisierbar sind. Die Vor- und Rücksprünge stellen zusätzlich eine Rotationssicherung dar.

Des Weiteren können durch Kontaktflächen definierte Kontaktebenen wenigstens eines Zwischenelements in einem von Null verschiedenen, insbesondere spitzen Winkel ε, zueinander stehen. Insbesondere können nur bei gekröpften Zwischenelementen unter einem Winkel ε zueinander stehende Kontaktebenen vorgesehen sein. Hierdurch wird ermöglicht, dass gekröpfte Zwischenelemente alternativ zu nicht gekröpften Zwischenelementen eingesetzt werden können, deren Kontaktebenen parallel zueinander verlaufen.

Das Zwischenelement ist über einen Zuganker zwischen dem ersten und dem zweiten Ansatz verspannbar. Hierdurch kann eine besonders feste und sichere Verbindung zwischen dem Zwischenelement und dem Basiselement bzw. der Hüftschale erreicht werden. Der Zuganker kann dabei insbesondere innerhalb des jeweiligen, beispielsweise als Hülse ausgebildeten Zwischenelements geführt und die beiden Enden des Zugankers können in dem Basiselement und der Hüftschale verankert sein.

Vorzugsweise sind zu verschieden langen Zwischenelementen in der Länge passende Zuganker vorgesehen.

Des Weiteren wird erfindungsgemäß vorgeschlagen, dass als Zuganker eine Dehnschraube vorgesehen ist.

Der Zuganker greift in einen im ersten Ansatz gehaltenen Kopf ein. Insbesondere kann der Kopf als Kugelgelenk ausgebildet sein. Hierdurch kann erreicht werden, dass der in dem ersten Ansatz gehaltene Kopf sowohl mit nicht gekröpften als auch mit gekröpften, insbesondere verschieden gekröpften Zwischenelementen zusammenwirken kann.

Es ist weiterhin bevorzugt, dass der Kopf über eine formschlüssige Steckverbindung zum Zwischenelement gegen Verdrehung gesichert ist. Dies ist insbesondere dann von Vorteil, wenn der Kopf in einer durch Drehung erreichbaren Stellung aus seiner Halterung in dem ersten Ansatz gelöst werden kann.

In einer weiteren bevorzugten Ausführungsform weist der Kopf über einen Winkelbereich γ von mehr als 180°, insbesondere von mehr als 200°, eine Kugelfläche auf, die eine in Form eines sich über den Pol hinweg erstreckenden Bandes ausgebildete Abflachung besitzt, wobei der Kopf in einer gegenüber der Funktionsstellung um 90° gedrehten Montagestellung in eine im ersten Ansatz ausgebildete Kavität einsetzbar ist.

Zur Verankerung der Beckenteilendoprothese am Darmbeinstumpf können in das Basiselement verschieden große Verankerungszapfen zur Befestigung des Basiselements am Darmbeinknochen einsetzbar sein, so dass in Abhängigkeit von den jeweiligen anatomischen Anforderungen ein passender Verankerungszapfen ausgewählt werden kann.

Des Weiteren kann in die Hüftschale eine Innenschale mit einer Schnappverbindung einsetzbar sein. Insbesondere kann in die Hüftschale eine Gelenkkugel zusammen mit einer Innenschale einsetzbar sein, welche die Gelenkkugel um mehr als 180° umschließt, so dass ein Oberschenkelknochen an der Beckenteilendoprothese befestigbar ist.

Um eine besonders einfache und effektive Halterung der Gelenkkugel zu erreichen, kann die Innenschale einen Haltering aufweisen, der nach dem Einsetzen einer Gelenkkugel an der Hüftschale befestigbar ist.

Als Teile eines Probierbaukastens können Basiselemente, Hüftschalen und Zwischenelemente vorgesehen sein, die jeweils aus einem sterilisierbaren Kunststoff bestehen und der Auffindung einer passenden räumlichen Stellung der Hüftschale relativ zum Basiselement dienen, wobei die Probierhüftschalen jeweils eine für eine Gelenkkugel passende Kontaktfläche aufweisen, so dass auf eine Innenschale verzichtet werden kann.

Die Prothesenteile für Probierprothesen und für endgültig einsetzbare Prothesen können unterschiedlich gekennzeichnet sein, um möglichen Verwechslungen vorzubeugen. Die Rasterstellungen von Zwischenelementen und ersten und zweiten Ansätzen können jeweils markiert sein, so dass die mittels der aus Kunststoff bestehenden Bauteile der Beckenteilendoprothese aufgefundene passende räumliche Stellung der Hüftschale auf einfache Weise auf die endgültig einsetzbaren Prothesen übertragbar ist.

Die Erfindung wird im Folgenden beispielhaft unter Bezugnahme auf die Zeichnung beschrieben. Es zeigen:
- Fig. 1 bis 3: verschiedene Ansichten einer erfindungsgemäßen Beckenteilendoprothese mit gerader Hülse,
- Fig. 4: einen vergrößerten Längsschnitt durch die Anordnung in Fig. 3,
- Fig. 5: eine perspektivische Ansicht einer erfindungsgemäßen Beckenteilendoprothese mit gekröpfter Hülse,
- Fig. 6: einen vergrößerten Längsschnitt durch die Anordnung in Fig. 5 mit eingesetzter Innenschale und Gelenkkugel,
- Fig. 7: eine perspektivische Ansicht einer erfindungsgemäßen Beckenteilendoprothese mit gerader Hülse und einer winkelförmigen, direkt am Sakrum ansetzbaren Verbindungsfläche,
- Fig. 8: einen vergrößerten Längsschnitt durch die Anordnung in Fig. 7, und
- Fig. 9 bis 12: verschiedene Ansichten eines Kugelgelenks einer erfindungsgemäßen Beckenteilendoprothese.

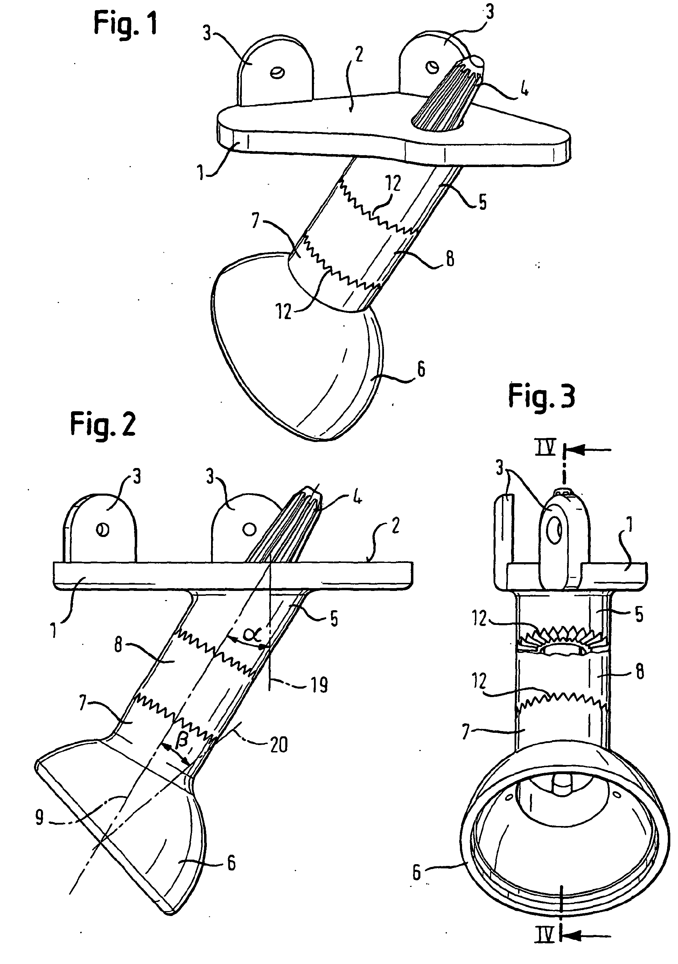

Die Fig. 1, 2 und 3 zeigen verschiedene Ansichten einer im zusammengesetzten Zustand dargestellten erfindungsgemäßen Beckenteilendoprothese zum Ersatz eines Beckenteils im Bereich des Hüftgelenks mit einem Basiselement 1, das an einem reserzierten Darmbeinknochen befestigbar ist, einer Hüftschale 6 zur Aufnahme einer Hüftgelenkkugel und einem als Hülse 8 ausgebildeten Zwischenelement, das zwischen Basiselement 1 und Hüftschale 6 angeordnet ist.

Das Basiselement 1 liegt mit einer ebenen Verbindungsfläche 2 an der Resektionsebene des nicht dargestellten reserzierten Darmbeinknochens an. Zur Befestigung des Basiselements 1 an dem Darmbeinknochen ist in das Basiselement 1 ein Verankerungszapfen 4 eingesetzt, der in eine in dem Darmbeinstumpf vorgesehene Bohrung eingreift.

Der Verankerungszapfen 4 ist gegenüber einer Orthogonalen 19 der Verbindungsfläche 2 um einen Winkel α geneigt. Der Verankerungszapfen 4 ist an seinem über die Verbindungsfläche 2 in Richtung des Darmbeinknochens vorstehenden Ende konisch zulaufend ausgebildet und weist eine längsgerippte Oberfläche auf.

Neben dem Verankerungszapfen 4 sind zur Befestigung des Basiselements 1 am Darmbeinknochen zwei Winkelstücke 3 vorgesehen, die in einem Randbereich des Basiselements 1 mit diesem verbunden sind und jeweils in einem Winkel von 90° von der Verbindungsfläche 2 des Basiselements 1 abstehen. Die Winkelstücke 3 sind jeweils über eine Durchgangsbohrung am Darmbeinknochen befestigbar.

Das Basiselement 1 weist ferner einen ebenfalls im Winkel α von der Orthogonalen 19 der ebenen Verbindungsfläche 2 wegstehenden ersten Ansatz 5 auf, so dass die Neigungen des ersten Ansatzes 5 und des Verankerungszapfens 4 gegenüber der Orthogonalen 19 der ebenen Verbindungsfläche 2 einander entsprechen.

Der erste Ansatz 5 des Basiselements 1 ist über die zylinderförmig ausgebildete Hülse 8 mit einem zweiten Ansatz 7 der kalottenförmig ausgebildeten Hüftschale 6 verbunden. Die jeweiligen Mittenachsen des Verankerungszapfens 4, des ersten Ansatzes 5, der Hülse 8 und des zweiten Ansatzes 7 bilden in den Fig. 1, 2 und 3 eine gemeinsame Mittenachse 9. Der zweite Ansatz 7 ist dabei um einen Winkel β gegenüber der Polachse 20 der Hüftschale 6 geneigt.

Die Hülse 8 und der erste und zweite Ansatz 5, 7 sind an ihren einander gegenüberliegenden Kontaktflächen jeweils mit Vor- und Rücksprüngen 12 versehen, die nach Art einer Hirth-Verzahnung ausgeführt sind, so dass bezüglich der Mittenachse 9 verschiedene Winkelstellungen der Hülse 8 gegenüber dem ersten Ansatz 5 und des zweiten Ansatzes 7 gegenüber der Hülse 8 möglich sind.

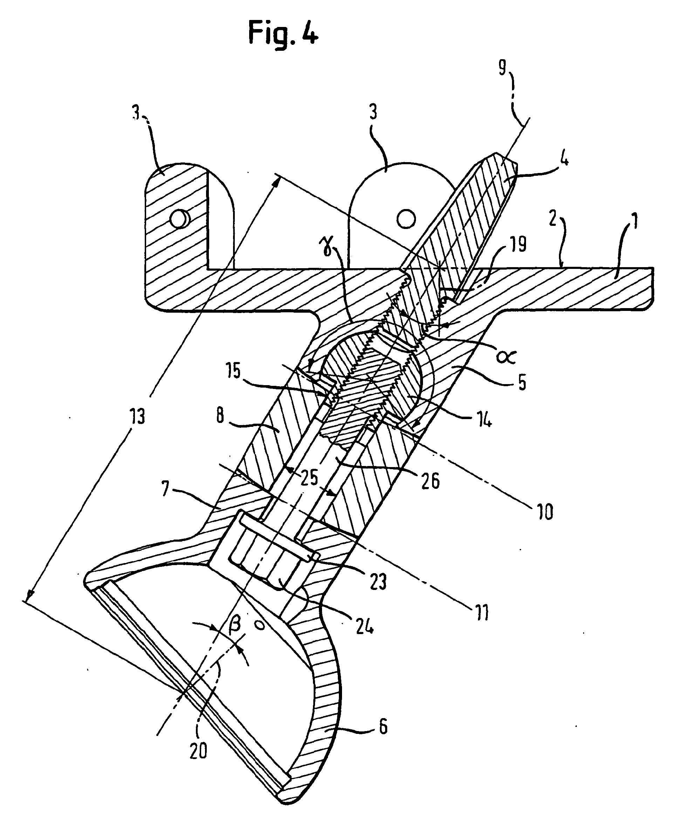

Fig. 4 zeigt einen Querschnitt der erfindungsgemäßen Beckenteilendoprothese entlang der Linie IV-IV in Fig. 3. Der Einfachheit halber wurde in Fig. 4 auf die Darstellung der Vor- und Rücksprünge 12 verzichtet. Aus Fig. 4 ist ersichtlich, dass die Kontaktflächen des ersten Ansatzes 5, der Hülse 8 und des zweiten Ansatzes 7 jeweils annähernd eben ausgebildet sind. Die im zusammengesetzten Zustand der Beckenteilendoprothese durch die Kontaktflächen definierten Kontaktebenen 10, 11 sind parallel zueinander ausgerichtet.

Fig. 4 kann weiterhin entnommen werden, dass sich der in das Basiselement 1 eingesetzte Verankerungszapfen 4 bis in den ersten Ansatz 5 des Basiselements 1 erstreckt und in das Basiselement 1 eingeschraubt ist. Im ersten Ansatz 5 des Basiselements 1 ist ferner eine Kavität ausgebildet, in der ein an anderer Stelle noch näher erläuterter Kugelkopf 14 gehalten ist, der über einen Winkelbereich γ von 200° eine Kugelfläche aufweist und über eine formschlüssige Steckverbindung 15 zur Hülse 8 gegen Verdrehung gesichert ist.

In den im ersten Ansatz 5 gehaltenen Kugelkopf 14 greift eine lang gestreckte, als Zuganker dienende Dehnschraube 26 ein, die die Hülse 8 zwischen dem ersten Ansatz 5 und dem zweiten Ansatz 7 verspannt. Der nicht in den Kugelkopf 14 eingeschraubte Teil der Dehnschraube 26 ist innerhalb der Hülse 8 bzw. innerhalb einer Bohrung des zweiten Ansatzes 7 der Hüftschale 6 angeordnet. Die Hülsen 8 weisen eine lichte Weite 25 auf, die größer ist als der Durchmesser der Dehnschraube 26.

Der Kopf der Dehnschraube 26 ist als Sechskant 24 ausgebildet, um das Einschrauben der Dehnschraube 26 in den im ersten Ansatz 5 gehaltenen Kugelkopf 14 zu ermöglichen. Die Dehnschraube 26 umfasst ferner einen Kragen 23, der im zusammengesetzten Zustand der Beckenteilendoprothese in einer mit der Bohrung verbundenen Kavität des zweiten Ansatzes 7 gehalten wird, so dass die Hülse 8 zwischen dem ersten Ansatz 5 und dem zweiten Ansatz 7 verspannbar ist.

Der Abstand 13 zwischen Basiselement 1 und Hüftschale 6 kann dadurch variiert werden, dass Hülsen 8 unterschiedlicher Länge und dazu in der Länge passende Dehnschrauben 26 vorgesehen sind, die jeweils zwischen dem ersten Ansatz 5 und dem zweiten Ansatz 7 einbaubar bzw. einschraubbar sind.

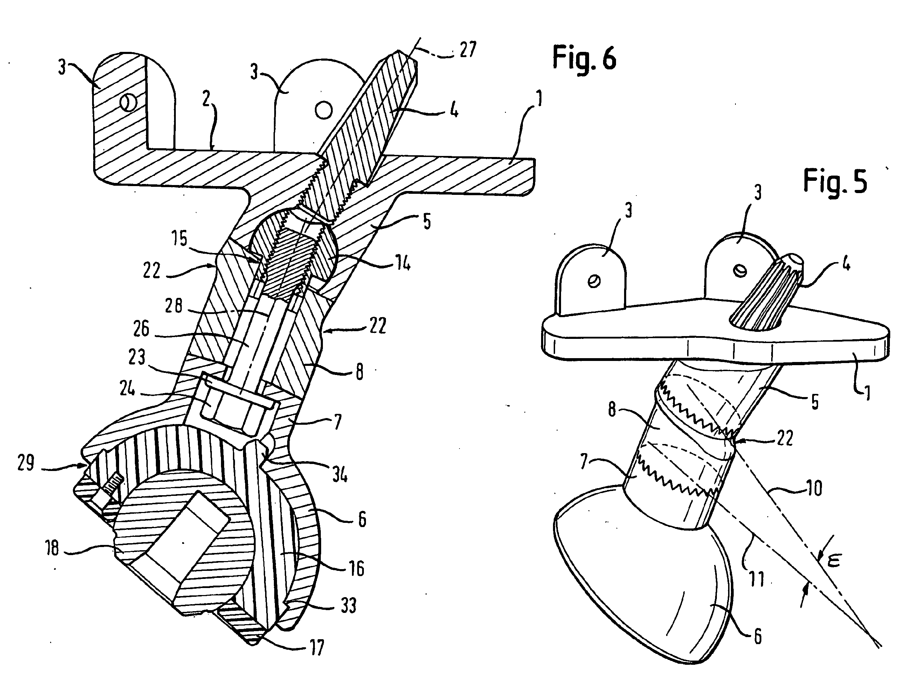

Die Fig. 5 und 6 zeigen eine Ausbildung einer erfindungsgemäßen Beckenteilendoprothese mit einer gekröpften Hülse 8, wobei jeweils gleiche oder entsprechende Teile mit denselben Bezugszeichen bezeichnet sind, so dass auf eine erneute Vorstellung verzichtet wird und lediglich die Abweichungen der in diesen Figuren dargestellten Ausführungsform gegenüber der ersten Ausführungsform erläutert werden.

Gemäß den Fig. 5 und 6 ist die Hülse 8 gekröpft bzw. hakenförmig gebogen. Die Kröpfung 22 der Hülse 8 hat zur Folge, dass die durch die Kontaktflächen definierten Kontaktebenen 10, 11 in einem spitzen Winkel ε zueinander stehen. Eine gemeinsame Mittenachse 27 des Verankerungszapfens 4 und des ersten Ansatzes 5 ist gegenüber einer gemeinsamen Mittenachse 28 der Dehnschraube 26, des größten Teils der Hülse 8 und des zweiten Ansatzes 7 geneigt. Aufgrund des Eingreifens der Dehnschraube 26 ist der in Fig. 6 dargestellte Kugelkopf 14 gegenüber dem in Fig. 4 gezeigten Kugelkopf verkippt.

In die Hüftschale 6 ist eine ebenfalls kalottenförmig ausgebildete Innenschale 16 eingesetzt, wobei die beiden jeweils eine Schulter 33 aufweisenden Schalen 6, 16 mittels einer Schnappverbindung 29 miteinander verbunden sind. Die Innenschale 16 umschließt dabei eine insbesondere künstliche Gelenkkugel 18 um mehr als 180°. Dazu umfasst die Innenschale 16 einen Haltering 17, der nach dem Einsetzen der Gelenkkugel 18 in die Innenschale 16 mit dieser verbunden wird.

Um eine korrekte Relativpositionierung der Innenschale 16 bezüglich der Hüftschale 6 zu gewährleisten, weist die Innenschale 16 in ihrem Polbereich eine in Richtung der Hüftschale 6 vorstehende Nase 34 auf, die im zusammengesetzten Zustand der Beckenteilendoprothese in eine die Kavität des zweiten Ansatzes 7 fortsetzende Ausnehmung der Hüftschale 6 eingreift.

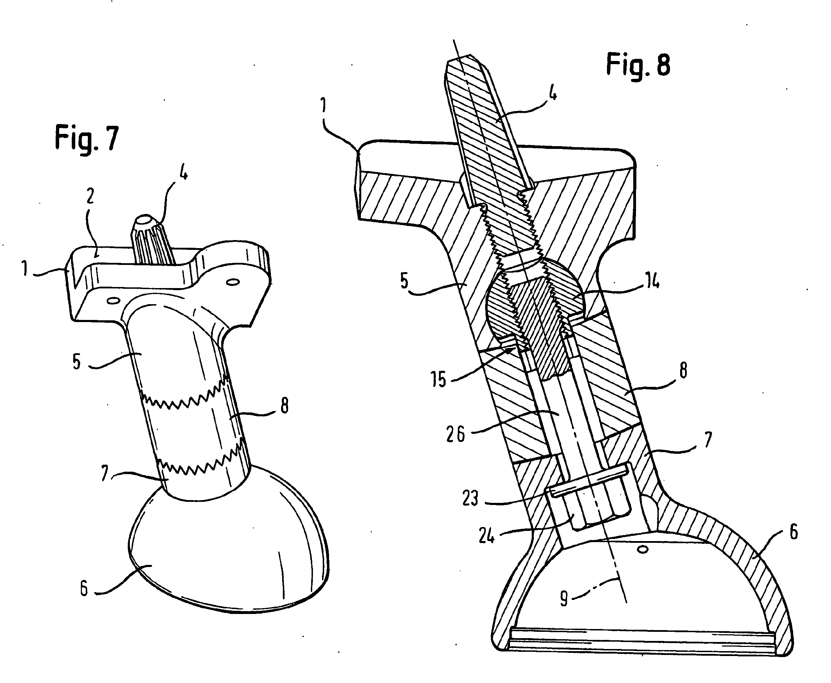

Die Fig. 7 und 8 zeigen eine weitere Ausführungsform einer erfindungsgemäßen Beckenteilendoprothese, die sich von den beiden vorstehend genannten Ausführungsformen insbesondere durch die Ausbildung des Basiselements 1 unterscheidet. Um ein Ansetzen direkt am Sakrum zu ermöglichen, weist die Beckenteilendoprothese gemäß den Fig. 7 und 8 eine winkelförmige Verbindungsfläche 2 auf.

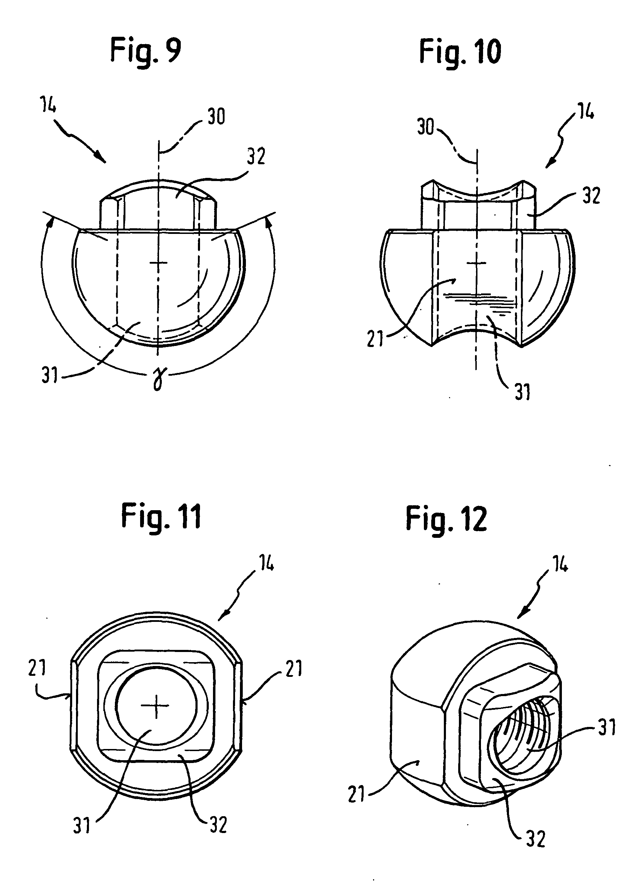

Die Fig. 9, 10, 11 und 12 zeigen verschiedene Ansichten des bereits vorstehend erwähnten Kugelkopfs 14. Der Kugelkopf 14 weist eine kugelige Grundform mit einer sich über einen Winkelbereich γ von 200° erstreckenden Kugelfläche auf. Der Kugelkopf weist weiterhin eine durchgehende, entlang der Polachse 30 des Kugelkopfs 14 ausgebildete Gewindebohrung 31 auf, in die im zusammengesetzten Zustand der Beckenteilendoprothese die Dehnschraube 26 eingreift.

An der dem Pol abgewandten Seite des Kugelkopfs 14 ist ein von der Bohrung 31 durchsetzter Steckvorsprung 32 ausgebildet. Der Steckvorsprung 32 ist in senkrecht zur Polachse 30 verlaufenden Ebenen im Wesentlichen quadratisch ausgebildet, wobei im zusammengesetzten Zustand der Beckenteilendoprothese der Steckvorsprung 32 in den entsprechend geformten Durchgang der Hülse 8 hineinragt, so dass der Kugelkopf 14 gegen Verdrehung gesichert ist.

Die Kugelfläche des Kugelkopfs 14 weist eine in Form eines sich über den Pol hinweg erstreckendes Bandes 21 ausgebildete Abflachung auf. In senkrecht zur Polachse 30 verlaufenden Ebenen besitzt der Kugelkopf 14 somit eine kreisrunde Form, die durch zwei gegenüberliegende, abgeflachte Seiten unterbrochen ist. Die Eingangsöffnung der Kavität des ersten Ansatzes 5 weist eine entsprechende Form auf, so dass der in die Kavität eingesetzte Kugelkopf 14 in einer gegenüber der Montagestellung um 90° gedrehten Funktionsstellung im ersten Ansatz 5 gehalten werden kann.

Nachfolgend wird der Zusammenbau einer erfindungsgemäßen Beckenteilendoprothese unter Bezugnahme auf die Figuren beschrieben.

Zunächst wird das Basiselement 1 an dem reserzierten Darmbeinknochen mittels des Verankerungszapfens 4 und der beiden Winkelstücke 3 befestigt, so dass die Verbindungsfläche 2 des Basiselements 1 an dem Darmbeinstumpf anliegt. Der Kugelkopf 14 wurde bereits vor dem Anbringen des Basiselements 1 an dem Darmbeinstumpf in die Kavität des ersten Ansatzes 5 eingesetzt, kann alternativ aber auch erst nach dem Anbringen des Basiselements 1 eingesetzt werden.

Danach wählt der Operateur aus einem Satz voneinander verschiedener Hülsen 8, die unterschiedliche Relativstellungen zwischen dem Basiselement 1 und der Hüftschale 6 ermöglichen, diejenige Hülse 8 aus, die die unter anatomischen Gesichtspunkten zweckmäßigste Relativstellung bewirkt. Die Hülsen 8 können unterschiedliche Längen aufweisen und/oder gekröpft ausgebildet sein.

Anschließend wird eine zu der Länge der ausgewählten Hülse 8 passende Dehnschraube 26 aus einem Satz unterschiedlich langer Dehnschrauben ausgewählt. Dann wird die Dehnschraube 26 durch die im zweiten Ansatz 7 der Hüftschale 6 ausgebildete Bohrung und durch die Hülse 8 hindurchgeführt und in die Bohrung 31 des im ersten Ansatz 5 gehaltenen Kugelkopfs 14 eingeschraubt.

Unter Berücksichtigung der Orientierung der Hüftschale 6 und ggf. der Kröpfung 22 der Hülse 8 wird die Dehnschraube 26 festgezogen, so dass die Vor- und Rücksprünge 12 der Kontaktflächen des ersten Ansatzes 5, der Hülse 8 und des zweiten Ansatzes 7 ineinander greifen. Der Steckvorsprung 32 des Kugelkopfs 14 greift in das Innere der Hülse 8 ein, so dass eine gegen Verdrehung gesicherte formschlüssige Steckverbindung 15 zur Hülse gebildet wird. Bei Verwendung einer gekröpften Hülse 8 wird der Kugelkopf 14 aufgrund des Eingreifens der Dehnschraube 26 entsprechend verkippt (Fig. 6).

Schließlich wird die Innenschale 16 in die Hüftschale 6 eingesetzt und mittels der Schnappverbindung 29 an dieser befestigt (Fig. 6). Zuvor wurde in die Innenschale 16 die Gelenkkugel 18 eingesetzt, die durch den Haltering 17 in der Innenschale 16 gehalten ist, wobei die den Haltering 17 umfassende Innenschale 16 die Gelenkkugel 18 um mehr als 180° umschließt.

Aufgrund der zur Verfügung stehenden, insbesondere verschieden langen und/oder gekröpften Zwischenelemente 8 besitzt eine erfindungsgemäße Beckenteilendoprothese eine erhöhte Flexibilität hinsichtlich der Positionierung und Ausrichtung der Hüftschale 6, so dass bei einem operativen Eingriff die jeweils aus anatomischen Gründen zweckmässigste Relativstellung zwischen Basiselement 1 und Hüftschlale 6 erreicht werden kann. Insbesondere werden dem Operateur größere Gestaltungsfreiheiten beim Setzen des Resektionsschnitts am Darmbeinknochen ermöglicht.

### Bezugszeichenliste

- 1: Basiselement
- 2: Verbindungsfläche
- 3: Winkelstück
- 4: Verankerungszapfen
- 5: erster Ansatz
- 6: Hüftschale
- 7: zweiter Ansatz
- 8: Hülse
- 9: Mittenachse
- 10: Kontaktebene
- 11: Kontaktebene
- 12: Vor- und Rücksprünge
- 13: Abstand
- 14: Kugelkopf
- 15: Steckverbindung
- 16: Innenschale
- 17: Haltering
- 18: Gelenkkugel
- 19: Orthogonale
- 20: Polachse
- 21: Band
- 22: Kröpfung
- 23: Kragen
- 24: Sechskant
- 25: lichte Weite
- 26: Dehnschraube
- 27: Mittenachse
- 28: Mittenachse
- 29: Schnappverbindung
- 30: Polachse
- 31: Bohrung
- 32: Steckvorsprung
- 33: Schulter
- 34: Nase

- α: Winkel
- β: Winkel
- γ: Winkel
- ε: Winkel

## Patentansprüche

1. Beckenteilendoprothese mit wenigstens einem an einem reserzierten Darmbeinknochen befestigbaren Basiselement (1) und zumindest einer in einem Abstand (13) dazu anbringbaren Hüftschale (6), wobei das Basiselement (1) einen ersten Ansatz (5) und die Hüftschale (6) einen zweiten Ansatz (7) aufweist,
**dadurch gekennzeichnet ,**
**dass** wenigstens ein separates Zwischenelement (8) vorgesehen ist, das zwischen dem ersten Ansatz (5) und dem zweiten Ansatz (7) einbaubar und über einen Zuganker (26), der in einen im ersten Ansatz (5) gehaltenen Kopf (14) eingreift, zwischen dem ersten und dem zweiten Ansatz (5, 7) verspannbar ist.

2. Beckenteilendoprothese nach Anspruch 1,
**dadurch gekennzeichnet ,**
**dass** mittels des Zwischenelements (8) unterschiedliche Relativstellungen zwischen dem Basislement (1) und der Hüftschale (6) realisierbar sind.

3. Beckenteilendoprothese nach Anspruch 1 oder 2,
**dadurch gekennzeichnet,**
**dass** wenigstens zwei voneinander verschiedene Zwischenelemente (8) vorgesehen sind, die jeweils zwischen dem ersten Ansatz (5) und dem zweiten Ansatz (7) einbaubar sind.

4. Beckenteilendoprothese nach einem der Ansprüche 1 bis 3,
**dadurch gekennzeichnet ,**
**dass** Zwischenelemente (8) unterschiedlicher Länge vorgesehen sind.

5. Beckenteilendoprothese nach einem der Ansprüche 1 bis 4,
**dadurch gekennzeichnet ,**
**dass** wenigstens ein Zwischenelement (8) gekröpft ist.

6. Beckenteilendoprothese nach einem der Ansprüche 1 bis 5,
**dadurch gekennzeichnet ,**
**dass** das Zwischenelement (8) und der erste und zweite Ansatz (5, 7) in Kontaktflächen jeweils mit Vor- und Rücksprüngen (12) versehen sind, die in verschiedenen Winkelstellungen einrastbar sind.

7. Beckenteilendoprothese nach Anspruch 6,
**dadurch gekennzeichnet ,**
**dass** die Vor- und Rücksprünge (12) in den Kontaktflächen einer Hirth-Verzahnung entsprechen.

8. Beckenteilendoprothese nach einem der Ansprüche 1 bis 7,
**dadurch gekennzeichnet ,**
**dass** durch Kontaktflächen definierte Kontaktebenen (10, 11) wenigstens eines Zwischenelements (8) in einem von Null verschiedenen, insbesondere spitzen Winkel ε, zueinander stehen.

9. Beckenteilendoprothese nach einem der Ansprüche 1 bis 8,
**dadurch gekennzeichnet ,**
**dass** der erste Ansatz (5) in einem Winkel α von der Orthogonalen (19) einer ebenen Verbindungsfläche (2) und/oder der zweite Ansatz (7) in einem Winkel β von der Polachse (20) der Hüftschale (6) wegsteht.

10. Beckenteilendoprothese nach einem der Ansprüche 1 bis 9,
**dadurch gekennzeichnet ,**
**dass** zu verschieden langen Zwischenelementen (8) in der Länge passende Zuganker (26) vorgesehen sind.

11. Beckenteilendoprothese nach einem der Ansprüche 1 bis 10,
**dadurch gekennzeichnet ,**
**dass** als Zuganker (26) eine Dehnschraube (26) vorgesehen ist.

12. Beckenteilendoprothese nach einem der Ansprüche 1 bis 11,
**dadurch gekennzeichnet ,**
**dass** der Kopf als Kugelgelenk (14) ausgebildet ist.

13. Beckenteilendoprothese nach einem der Ansprüche 1 bis 12 ,
**dadurch gekennzeichnet ,**
**dass** der Kopf (14) über eine formschlüssige Steckverbindung (15) zum Zwischenelement (8) gegen Verdrehung gesichert ist.

14. Beckenteilendoprothese nach einem der Ansprüche 1 bis 13,
**dadurch gekennzeichnet ,**
**dass** der Kopf (14) über einen Winkelbereich γ von mehr als 180°, insbesondere von mehr als 200°, eine Kugelfläche aufweist, die eine in Form eines sich über den Pol hinweg erstreckenden Bandes (21) ausgebildete Abflachung besitzt, wobei der Kopf (14) in einer gegenüber der Funktionsstellung um 90° gedrehten Montagestellung in eine im ersten Ansatz (5) ausgebildete Kavität einsetzbar ist.

15. Beckenteilendoprothese nach einem der Ansprüche 1 bis 14,
**dadurch gekennzeichnet ,**
**dass** in das Basiselement (1) verschieden große Verankerungszapfen (4) zur Befestigung des Basiselements (1) am Darmbeinknochen einsetzbar sind.

16. Beckenteilendoprothese nach einem der Ansprüche 1 bis 15,
**dadurch gekennzeichnet ,**
**dass** in die Hüftschale (6) eine Innenschale (16) mit einer Schnappverbindung (29) einsetzbar ist.

17. Beckenteilendoprothese nach einem der Ansprüche 1 bis 16,
**dadurch gekennzeichnet ,**
**dass** in die Hüftschale (6) eine Gelenkkugel (18) zusammen mit einer Innenschale (16) einsetzbar ist, welche die Gelenkkugel (18) um mehr als 180° umschließt.

18. Beckenteilendoprothese nach Anspruch 16 oder 17,
**dadurch gekennzeichnet ,**
**dass** die Innenschale (16) einen Haltering (17) aufweist, der nach dem Einsetzen einer Gelenkkugel (18) an der Hüftschale (6) befestigbar ist.

19. Beckenteilendoprothese nach einem der Ansprüche 1 bis 18,
**dadurch gekennzeichnet ,**
**dass** als Teile eines Probierbaukastens Basiselemente, Hüftschalen und Zwischenelemente vorgesehen sind, die jeweils aus einem sterilisierbaren Kunststoff bestehen und der Auffindung einer passenden räumlichen Stellung der Hüftschale relativ zum Basiselement dienen, wobei die Probierhüftschalen jeweils eine für eine Gelenkkugel passende Kontaktfläche aufweisen.

20. Beckenteilendoprothese nach Anspruch 19,
**dadurch gekennzeichnet ,**
**dass** die Prothesenteile für Probierprothesen und für endgültig einsetzbare Prothesen (1, 6, 8) unterschiedlich **gekennzeichnet** sind.

21. Beckenteilendoprothese nach Anspruch 19 oder 20,
**dadurch gekennzeichnet ,**
**dass** die Rasterstellungen von Zwischenelementen (8) und ersten und zweiten Ansätzen (5, 7) jeweils markiert sind.

## Claims

1. An endoprosthesis for part of the pelvis having at least one base element (1) which can be secured to a resected iliac bone and at least one hip shell (6) attachable at a spacing (13) thereto, with the base element (1) having a first neck (5) and the hip shell (6) having a second neck (7),
**characterized in that**
at least one separate intermediate element (8) is provided which can be installed between the first neck (5) and the second neck (7) and can be clamped between the first and the second necks (5, 7) via a draw anchor (26) which engages into a head (14) held in the first neck (5).

2. An endoprosthesis for part of the pelvis in accordance with claim 1, **characterized in that** different relative positions between the base element (1) and the hip shell (6) can be realized by means of the intermediate element (8).

3. An endoprosthesis for part of the pelvis in accordance with claim 1 or claim 2, **characterized in that** at least two intermediate elements (8) are provided which differ from one another and which can each be installed between the first neck (5) and the second neck (7).

4. An endoprosthesis for part of the pelvis in accordance with any one of the claims 1 to 3, **characterized in that** intermediate elements (8) of different lengths are provided.

5. An endoprosthesis for part of the pelvis in accordance with any one of the claims 1 to 4, **characterized in that** at least one intermediate element (8) is cranked.

6. An endoprosthesis for part of the pelvis in accordance with any one of the claims 1 to 5, **characterized in that** the intermediate element (8) and the first and second necks (5, 7) are each provided in contact areas with projections and recesses (12) latchable at different angular positions.

7. An endoprosthesis for part of the pelvis in accordance with claim 6, **characterized in that** the projections and recesses (12) in the contact areas correspond to a toothed Hirth coupling.

8. An endoprosthesis for part of the pelvis in accordance with any one of the claims 1 to 7, **characterized in that** contact planes (10, 11) of at least one intermediate element (8) and defined by contact areas stand at an angle to one another, in particular an acute angle ε, different from zero.

9. An endoprosthesis for part of the pelvis in accordance with any one of the claims 1 to 8, **characterized in that** the first neck (5) projects from the orthogonal (19) of a planar connection surface (2) and an angle α and/or **in that** the second neck (7) projects from the polar axis (20) of the hip shell (6) at an angle β.

10. An endoprosthesis for part of the pelvis in accordance with any one of the claims 1 to 9, **characterized in that** draw anchors (26) of matching length are provided for intermediate elements (8) of different lengths.

11. An endoprosthesis for part of the pelvis in accordance with any one of the claims 1 to 10, **characterized in that** a waisted bolt (26) is provided as the draw anchor (26).

12. An endoprosthesis for part of the pelvis in accordance with any one of the claims 1 to 11, **characterized in that** the head is made as a ball joint (14).

13. An endoprosthesis for part of the pelvis in accordance with any one of the claims 1 to 12, **characterized in that** the head (14) is secured against rotation with respect to the intermediate element (8) via a shape-matched plug-in connection (15).

14. An endoprosthesis for part of the pelvis in accordance with any one of the claims 1 to 13, **characterized in that** the head (14) has a spherical surface over an angular range γ of more than 180°, in particular of more than 200°, the spherical surfacing having a flattened section shaped in the form of a band (21) extending beyond the pole, with the head (14) being able to be inserted into a cavity formed in the first neck (5) in an installation position rotated by 90° with respect to the functional position.

15. An endoprosthesis for part of the pelvis in accordance with any one of the claims 1 to 14, **characterized in that** differently sized anchoring spigots (4) can be inserted into the base element (1) for the securing of the base element (1) to the iliac bone.

16. An endoprosthesis for part of the pelvis in accordance with any one of the claims 1 to 15, **characterized in that** an inner shell (16) can be inserted into the hip shell (6) with a snap connection (29).

17. An endoprosthesis for part of the pelvis in accordance with any one of the claims 1 to 16, **characterized in that** a joint ball (18) can be inserted into the hip shell (6) together with an inner shell (16) which surrounds the joint ball (18) by more than 180°.

18. An endoprosthesis for part of the pelvis in accordance with claim 16 or claim 17, **characterized in that** the inner shell (16) has a holding ring (17) which can be secured to the hip shell (6) after the insertion of a joint ball (18).

19. An endoprosthesis for part of the pelvis in accordance with any one of the claims 1 to 18, **characterized in that** base elements, hip shells and intermediate elements are provided as parts of a trial kit which each consist of a plastic, which can be sterilized, and which serve for the location of a matching spatial position of the hip shell relative to the base element, with the trial hip shells each having a contact area matching a joint ball.

20. An endoprosthesis for part of the pelvis in accordance with claim 19, **characterized in that** the prosthesis parts for trial prostheses and for finally insertable prostheses (1, 6, 8) are **characterized** differently.

21. An endoprosthesis for part of the pelvis in accordance with claim 19 or claim 20, **characterized in that** the engagement positions of intermediate elements (8) and of first and second noses (5, 7) are respectively marked.

## Revendications

1. Endoprothèse partielle de bassin, comportant au moins un élément de base (1) qui peut être fixé sur un os iliaque réséqué et au moins une coque de hanche (6) qui peut être appliquée à une distance (13) de celui-ci, l'élément de base (1) présentant une première embase (5) et la coque de hanche (6) présentant une deuxième embase (7),
**caractérisée en ce qu'**il est prévu au moins un élément intermédiaire (8) séparé qui peut être monté entre la première embase (5) et la deuxième embase (7) et qui peut être serré entre la première embase (5) et la deuxième embase (7) via un tirant d'ancrage (26) qui s'engage dans une tête (14) retenue dans la première embase (5).

2. Endoprothèse partielle de bassin selon la revendication 1, **caractérisée en ce qu'**au moyen de l'élément intermédiaire (8) peuvent être réalisées différentes positions relatives entre l'élément de base (1) et la coque de hanche (6).

3. Endoprothèse partielle de bassin selon la revendication 1 ou 2, **caractérisée en ce qu'**il est prévu au moins deux éléments intermédiaires (8) différents l'un de l'autre qui peuvent être chacun montés entre la première embase (5) et la deuxième embase (7).

4. Endoprothèse partielle de bassin selon l'une des revendications 1 à 3, **caractérisée en ce qu'**il est prévu des éléments intermédiaires (8) de différente longueur.

5. Endoprothèse partielle de bassin selon l'une des revendications 1 à 4, **caractérisée en ce qu'**au moins un élément intermédiaire (8) est coudé.

6. Endoprothèse partielle de bassin selon l'une des revendications 1 à 5, **caractérisée en ce que** l'élément intermédiaire (8) et la première embase (5) et la deuxième embase (7) sont pourvues de saillies et retraits (12) respectifs dans des surfaces de contact, qui peuvent s'enclencher dans différentes positions angulaires.

7. Endoprothèse partielle de bassin selon la revendication 6, **caractérisée en ce que** les saillies et retraits (12) dans les surfaces de contact correspondent à un crantage Hirth.

8. Endoprothèse partielle de bassin selon l'une des revendications 1 à 7, **caractérisée en ce que** des plans de contact (10, 11), définis par des surfaces de contact, d'au moins un élément intermédiaire (8) se trouvent l'un par rapport à l'autre sous un angle différent de zéro, en particulier sous un angle aigu ε.

9. Endoprothèse partielle de bassin selon l'une des revendications 1 à 8, **caractérisée en ce que** la première embase (5) s'écarte sous un angle α de l'orthogonale (19) d'une surface de liaison (2) plane et/ou la deuxième embase (7) s'écarte sous un angle β de l'axe polaire (20) de la coque de hanche (6).

10. Endoprothèse partielle de bassin selon l'une des revendications 1 à 9, **caractérisée en ce qu'**il est prévu des tirants d'ancrage (26) adaptés en longueur à des éléments intermédiaires (8) de différentes longueurs.

11. Endoprothèse partielle de bassin selon l'une des revendications 1 à 10, **caractérisée en ce qu'**il est prévu une vis d'extension (26) en tant que tirant d'ancrage (26).

12. Endoprothèse partielle de bassin selon l'une des revendications 1 à 11, **caractérisée en ce que** la tête est réalisée sous forme d'articulation sphérique (14).

13. Endoprothèse partielle de bassin selon l'une des revendications 1 à 12, **caractérisée en ce que** la tête (14) est bloquée à l'encontre d'une rotation par une liaison par enfichage (15) à coopération de formes par rapport à l'élément intermédiaire (8).

14. Endoprothèse partielle de bassin selon l'une des revendications 1 à 13, **caractérisée en ce que** la tête (14) présente sur une plage angulaire γ de plus de 180°, en particulier de plus de 200°, une surface sphérique qui possède un aplatissement réalisé sous forme d'un ruban (21) s'étendant au-delà du pôle, la tête (14) pouvant être mise en place dans une cavité réalisée dans la première embase (5) dans une position de montage tournée de 90° par rapport à la position fonctionnelle.

15. Endoprothèse partielle de bassin selon l'une des revendications 1 à 14, **caractérisée en ce que** des tenons d'ancrage (4) de différentes tailles peuvent être mis en place dans l'élément de base (1) pour fixer l'élément de base (1) sur l'os iliaque.

16. Endoprothèse partielle de bassin selon l'une des revendications 1 à 15, **caractérisée en ce qu'**une coque intérieure (16) peut être mise en place dans la coque de hanche (6) avec une liaison par enclenchement (29).

17. Endoprothèse partielle de bassin selon l'une des revendications 1 à 16, **caractérisée en ce qu'**une sphère d'articulation (18) peut être mise en place dans la coque de hanche (6) conjointement avec une coque intérieure (16) qui entoure la sphère d'articulation (18) sur plus de 180°.

18. Endoprothèse partielle de bassin la revendication 16 ou 17, **caractérisée en ce que** la coque intérieure (16) présente un anneau de retenue (17) qui, après la mise en place d'une sphère d'articulation (18), peut être fixé sur la coque de hanche (6).

19. Endoprothèse partielle de bassin selon l'une des revendications 1 à 18, **caractérisée en ce qu'**en tant que pièces d'un système modulaire d'essai sont prévus des éléments de base, des coques de hanche et des éléments intermédiaires qui sont chacun en une matière plastique stérilisable et qui servent à trouver une position dans l'espace de la coque de hanche qui est adaptée par rapport à l'élément de base, les coques de hanche d'essai présentant chacune une surface de contact adaptée à une sphère d'articulation.

20. Endoprothèse partielle de bassin selon la revendication 19, **caractérisée en ce que** les parties de prothèse pour des prothèses d'essai et pour des prothèses (1, 6, 8) qui peuvent être définitivement mises en place sont marquées différemment.

21. Endoprothèse partielle de bassin selon la revendication 19 ou 20, **caractérisée en ce que** les positions échelonnées entre les éléments intermédiaires (8) et les première et deuxième embases (5, 7) sont chacune marquées.
